Europäisches Patentamt

European Patent Office

Office européen des brevets

(11) Veröffentlichungsnummer: **0 267 519 B1**

(12)

# EUROPÄISCHE PATENTSCHRIFT

(45) Veröffentlichungstag der Patentschrift: **15.01.92**

(21) Anmeldenummer: **87116134.5**

(22) Anmeldetag: **03.11.87**

(51) Int. Cl.5: **G01N 33/52**, C12Q 1/00, //G01N33/94,C12Q1/50

(54) **Testträger zur analytischen Bestimmung eines Bestandteils einer Körperflüssigkeit.**

(30) Priorität: **12.11.86 DE 3638654**

(43) Veröffentlichungstag der Anmeldung:
**18.05.88 Patentblatt 88/20**

(45) Bekanntmachung des Hinweises auf die
Patenterteilung:
**15.01.92 Patentblatt 92/03**

(84) Benannte Vertragsstaaten:
**AT BE CH DE ES FR GB GR IT LI LU NL SE**

(56) Entgegenhaltungen:
**EP-A- 0 133 895**
**EP-A- 0 182 373**
**US-A- 3 936 357**

(73) Patentinhaber: **BOEHRINGER MANNHEIM GMBH**
**Sandhofer Strasse 116**
**W-6800 Mannheim 31(DE)**

(72) Erfinder: **Freitag, Helmut, Dr.rer.nat**
**9115 Hague Road**
**Indianapolis, IN 46250(US)**
Erfinder: **Steinbiss, Joachim, Dr.**
**Lortzingstr. 14**
**W-6100 Darmstadt 23(DE)**
Erfinder: **Rothe, Anselm, Dr.rer.nat.**
**Tiefenklinger Weg 21**
**W-6943 Birkenau(DE)**

EP 0 267 519 B1

## Beschreibung

Die Erfindung betrifft einen Testträger zur analytischen Bestimmung eines Bestandteils einer Körperflüssigkeit mit einer Basisschicht und mindestens zwei flachen im Ausgangszustand des Testträgers vor Durchführung einer Bestimmung voneinander getrennten, jedoch durch externe Manipulation in Kontakt zueinander bringbaren Testschichten.

Während früher im klinischen Labor die Konzentration beispielsweise der Bestandteile des Blutes praktisch ausschließlich mit Hilfe flüssiger Reagenzien bestimmt wurde, haben in jüngerer Zeit sogenannte trägergebundene Tests zunehmend an Bedeutung gewonnen. Bei diesen sind die Reagenzien in entsprechenden Schichten eines festen Testträgers eingebettet, auf den ein Tropfen der Probe aufgebracht wird. Die Reaktion von Probe und Reagenzien führt zu einem nachweisbaren Signal, insbesondere einem Farbumschlag, welcher visuell oder mit Hilfe eines Gerätes, meistens reflexionsphotometrisch, ausgewertet werden kann.

Testträger sind häufig als Teststreifen ausgebildet, die im wesentlichen aus einer länglichen Basisschicht aus Kunststoffmaterial und darauf angebrachten Testfeldern bestehen. Es sind jedoch auch Testträger bekannt, die als quadratische oder rechteckige Plättchen gestaltet sind.

Die meisten Verfahren der Klinischen Chemie erfordern eine präzise auszuführende Folge einzelner Reaktionsschritte. Hierzu gehört beispielsweise die Serum- oder Plasmagewinnung, die Entfernung von Störsubstanzen und schließlich die Durchführung von einem oder mehreren Nachweisschritten. Bei jedem dieser Schritte müssen im allgemeinen zu einem bestimmten Zeitpunkt Reagenzien zugegeben und eine genaue Reaktionszeit eingehalten werden. Besonders bei den immunchemischen Verfahren sind in aller Regel mehrere präzise aufeinander abgestimmte Reaktionsschritte notwendig.

Testträger hatten in der Anfangszeit der Entwicklung nur eine einzige Testschicht. Dort wurden zwar verschiedene Reagenzien derartig kombiniert, daß auch komplizierte Reaktionsfolgen möglich waren, jedoch ließen einschichtige Testträger kein zeitlich definiertes Ablaufen mehrerer hintereinander geschalteter Reaktionen zu. Ähnliches gilt auch für Testträger, bei denen mehrere Testschichten in einem einen Flüssigkeitsaustausch ermöglichenden Kontakt zueinander stehen. Zwar dringt hier die Probe nach und nach von Schicht zu Schicht durch den Testträger. Eine präzise Definition der Kontaktzeiten zwischen den verschiedenen Testschichten ist aber auch hier nicht möglich.

Dies hat zur Entwicklung der eingangs erwähnten Testträger geführt, bei denen mindestens zwei Testschichten im Ausgangszustand, d. h. vor Durchführung einer analytischen Bestimmung voneinander getrennt sind, jedoch so angeordnet, sind, daß sie durch externe Manipulation, beispielsweise manuell in Kontakt miteinander gebracht werden können. Derartige Testträger sind beispielsweise im US-Patent 3 933 594 und im Deutschen Patent 3130749 beschrieben. In diesen Fällen befindet sich eine Testschicht auf dem Basisträger, eine weitere Testschicht ist mit einer Kante neben der erstgenannten Testschicht auf dem Basisträger befestigt. Sie steht im Ausgangszustand schräg von Basisträger ab, so daß sie die erstgenannte Testschicht nicht berührt. Durch äußeren Druck läßt sie sich wie eine Klappe auf die auf der Basisschicht befestigte Testschicht herunterdrücken, so daß in diesem Moment ein Flüssigkeitsübertritt von der einen Testschicht in die andere Testschicht möglich ist. Dieser Testaufbau ist verhältnismäßig einfach, erlaubt aber nur einen zweistufigen Reaktionsablauf.

In der US-Patentschrift 3 936 357 ist ein Testträger beschrieben, der einen mehrstufigen Testablauf ermöglicht. Er besitzt mehrere Testschichten, die durch Scharniere verbunden sind und die durch Knicken und Umklappen wechselseitig miteinander in Kontakt gebracht werden können. Dieser Testträger ist jedoch kompliziert aufgebaut und erfordert eine komplizierte Handhabung, so daß er sich nicht gut für die Auswertung mit einem Meßgerät eignet. Aufgrund des komplizierten Aufbaus ist die Herstellung außerordentlich aufwendig. Schließlich muß zur Einleitung eines Reaktionsschritts jeweils Druck auf die Testschichten ausgeübt werden. Dies ist nicht mit allen Testschichten möglich.

Hiervon ausgehend liegt der vorliegenden Erfindung das Problem zugrunde, eine verbesserte Möglichkeit zu schaffen, den Reaktionsablauf auf einem Testträger zu unterbrechen und zu einem beliebigen Zeitpunkt freizugeben, so daß ein zeitlich definierter Reaktionsablauf von mindestens zwei, bevorzugt mindestens drei Reaktionsschritten möglich ist. Der Testträger soll geeignet sein für die Auswertung mit einem entsprechenden Gerät mit dem er ein System bildet.

Diese Aufgabe wird bei einem Testträger der eingangs bezeichneten Art dadurch gelöst, daß eine erste Testschicht und eine zweite Testschicht im wesentlichen nebeneinander im Ausgangszustand durch einen Spalt getrennt auf der Basisschicht angeordnet sind und daß ein Schalterelement vorgesehen ist, welches aus einem kapillaraktiven Material besteht. Das Schalterelement ist so dimensioniert, daß es den Spalt überbrücken kann und es ist so gelagert und angeordnet, daß es in einer Position mindestens eine der Testschichten nicht kontaktiert, daß es jedoch durch externen mechanischen Druck in eine zweite Position gebracht werden kann, in der es beide Testschichten derartig kontaktiert, daß ein Flüssigkeitsaustausch zwischen den Testschichten möglich ist.

Eine Testschicht im Sinne der Erfindung ist jede in irgendeiner Weise am Testablauf beteiligte Schicht eines Testträgers. Mindestens zwei Testschichten sind beim erfindungsgemäßen Testträger im wesentlichen nebeneinander auf der Basisschicht derart angeordnet, daß sie im Ausgangszustand (also vor Durchführung einer analytischen Bestimmung) durch einen Spalt getrennt sind. Im wesentlichen nebeneinander ist dahingehend zu verstehen, daß die Testschichten sich über den bei weitem größten Teil ihrer Fläche nicht überlappen. Falls sie sich überhaupt nicht überlappen verläuft der Spalt senkrecht zur Basisschicht zwischen den Testschichten. Die Erfindung umfaßt jedoch auch Lösungen, bei denen sich die Testschichten geringfügig überlappen, die überlappenden Bereiche jedoch durch einen im wesentlichen parallel zur Basisschicht verlaufenden Spalt getrennt sind.

Das Schalterelement besteht aus einem kapillaraktiven Material, d. h. einem Material, in dem eine Flüssigkeit durch Kapillarwirkung transportiert werden kann. Geeignet sind insbesondere Gewebe, Gewirke, Vliese oder Papiere, also aus Fasern bestehende Strukturen, bei denen die Flüssigkeit durch die zwischen den Fasern bestehenden Hohlräume transportiert wird. Es können aber auch andere poröse Strukturen eingesetzt werden. Derartige Stukturen sind beispielsweise in dem US-Patent 3992158 als sogenannte Ausbreitungsschichten beschrieben.

Die gewünschte Lagerung und Anordnung des Schalterelements an dem Testträger kann in verschiedenerlei Weise realisiert werden. So kann es beispielsweise permanent mit einer der Testschichten in Verbindung stehen, so daß ein Flüssigkeitsaustausch zwischen dieser Testschicht und dem Schalterelement jederzeit möglich ist. Insbesondere kann es in diesem Fall auch ein integraler Bestandteil dieser Testschicht sein.

Gemäß einer bevorzugten Ausführungsform ist eine biegbare Deckfolie vorgesehen, die eine der Testschichten klappenartig überspannt. Sie ist auf der von der anderen Testschicht abgewandten Seite an der Basisschicht scharnierartig befestigt (insoweit ähnlich dem US-Patent 3 933 594). Auf der der anderen Testschicht zugewandten Seite erstreckt sich die Deckfolie so weit, daß sie das Schalterelement zumindest teilweise überlappt und abdeckt, wenn sie durch externen Druck auf die Basisschicht zubewegt wird. Dadurch wird auch auf das Schalterelement ein Druck ausgeübt, d. h. der Schalter kann mittels der Deckfolie betätigt werden. Dadurch wird eine Verschmutzung des Geräteteils, mit welchem die klappenartige Deckfolie nach unten gedrückt wird, vermieden. Diese Ausführungsform ist besonders vorteilhaft, wenn der Meßkopf eines Reflexionsphotometers, welches zur Auswertung des Testträgers eingesetzt wird und mit diesem ein System bildet zugleich zum Andrücken der Deckfolie verwendet wird. Ein derartiges Gerät ist in der Europäischen Patentanmeldung mit der Publikationshummer 129 220 beschrieben.

Eine weitere vorteilhafte Weiterbildung der Erfindung sieht vor, daß zwischen der Deckfolie und der von ihr überspannten Testschicht eine dritte Testschicht derartig angeordnet ist, daß sie nur durch externen Druck auf die Deckfolie in einen einen Flüssigkeitsaustausch ermöglichenden Kontakt mit der von der Deckfolie überspannten Testschicht gebracht werden kann. Diese dritte Testschicht kann beispielsweise unmittelbar auf die Unterseite der Deckfolie beschichtet sein. Es kann jedoch auch eine separate Testschicht zwischen der Deckfolie und der von dieser überspannten Testschicht vorgesehen sein.

Bevorzugt sind die Materialien der Testschicht und des Schalterelementes (zumindest an den sich gegenseitig kontaktierenden Oberflächen) so aufeinander abgestimmt, daß das Schalterelement im feuchten Zustand an der Testschicht haftet. Sobald das Schalterelement mit einer von der Probenflüssigkeit durchfeuchteten Testschicht in Kontakt gebracht wird, saugt sich aufgrund seiner kapillaraktiven Eigenschaften voll und wird selbst feucht. Wenn es nun gemäß dieser bevorzugten Ausführungsform an der Testschicht haftet, bleibt die Flüssigkeitsverbindung zwischen den Testschichten bestehen, auch wenn der externe Druck, mit dem das Schalterelement in einen einen Flüssigkeitsaustausch ermöglichenden Kontakt zwischen den Testschichten gebracht wird, wieder aufgehoben wird. Diese Ausführungsform ist, wie im folgenden noch näher beschrieben wird, besonders vorteilhaft im Zusammenhang mit der zuvor beschriebenen Ausführungsform mit einer Deckfolie und einer unter der Deckfolie befindlichen dritten Testschicht.

Die Erfindung wird im folgenden anhand von in den Figuren schematisch dargestellten Ausführungsbeispielen näher erläutert.

Es zeigen:

Figur 1      einen Querschnitt eines erfindungsgemäßen Testträgers, wobei sich das Schalterelement in der ersten Position (Schalter geöffnet) befindet.

Figur 2      den Testträger nach Figur 1, wobei sich das Schalterelement in der zweiten Position (Schalter geschlossen) befindet.

Figur 3 - 5      drei weitere verschiedene Ausführungsformen eines erfindungsgemäßen Testträgers im Querschnitt.

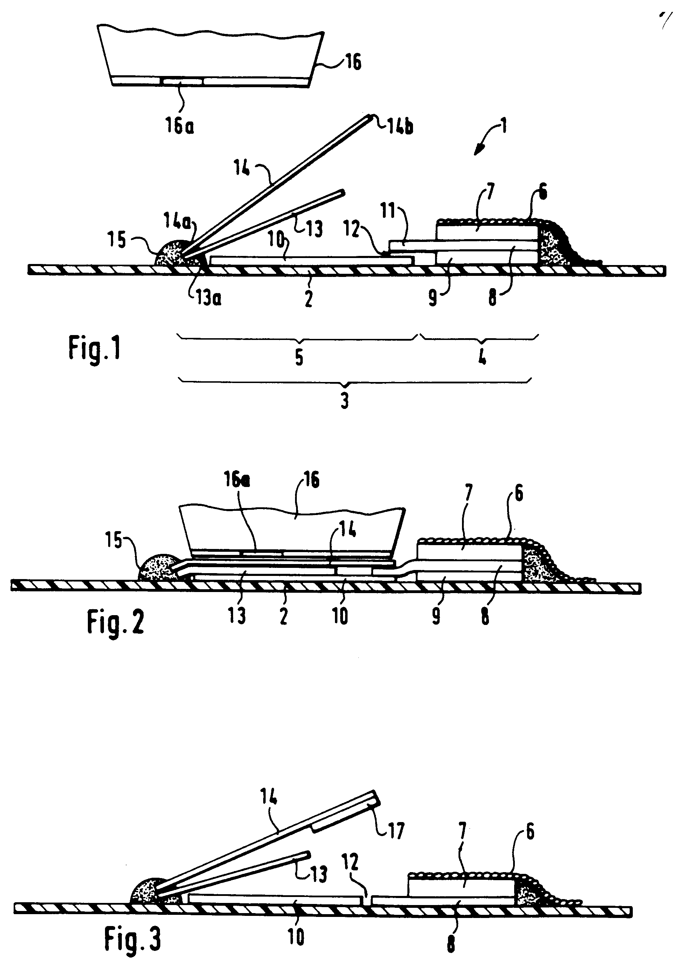

Der in Figur 1 dargestellte Testträger 1 hat die prinzipielle Form eines Teststreifens. Es handelt sich jedoch um ein mit den früheren Teststreifen kaum mehr vergleichbares hochwertiges Analysesystem. Der Testträger ist - abgesehen von den Besonderheiten der jeweiligen Erfindung - ähnlich dem in der deutschen Patentanmeldung 3523439 beschriebenen Testträger.

Auf einer Basisschicht 2 befindet sich der insgesamt mit 3 bezeichnete eigentliche Testbereich, der sich nur über einen Teil der Länge der Basisschicht 2 erstreckt. Der Testbereich 3 läßt sich in eine Probenaufgabezone 4 und in eine Auswertezone 5 unterteilen.

In der Probenaufgabezone erkennt man von oben nach unten ein Abdecknetz 6, eine Erythrozytenabtrennschicht 7, eine erste Testschicht 8 und eine Stützschicht 9.

Eine zweite Testschicht 10 ist im wesentlichen in der Auswertezone 5 auf der Basisschicht 2 angeordnet. Die erste Testschicht 8 und die zweite Testschicht 10 liegen im wesentlichen nebeneinander, überlappen jedoch bei der dargestellten Ausführungsform geringfügig. Bevorzugt überlappen beide Schichten um höchstens etwa 30 %. Der die Testschicht 10 überlappende Bereich der Testschicht 8 bildet das Schalterelement 11. Das Schalterelement 11 ist bei der hier dargestellten Ausführungsform also ein integraler Bestandteil der Testschicht 8. Dadurch, daß die erste Testschicht 8 auf der Stützschicht 9 gelagert ist, hat das Schalterelement 11, welches die Testschicht 8 in einer Ebene fortsetzt, einen geringfügigen Abstand von der Testschicht 10, d. h. zwischen dem Schalterelement 11 und der Testschicht 10 verläuft ein Spalt 12. Das Material der Testschicht 8 und des Schalterelements 11 ist ausreichend steif, daß der Abstand zwischen Schalterelement 11 und Testschicht 10 erhalten bleibt, solange kein externer mechanischer Druck von oben auf das Schalterelement 11 ausgeübt wird.

Über der zweiten Testschicht 10 sind in der Auswertezone eine dritte Testschicht 13 und eine Deckfolie 14 angeordnet. Beide sind an einer Kante 13 a bzw. 14 a mit einem Schmelzkleberstreifen 15 an der Basisschicht 2 befestigt. Sie sind so angeklebt, daß sie schräg von der Basisschicht 2 weg verlaufen. Die Testschicht 10 kommt ohne Anwendung äußeren Drucks nicht mit der dritten Testschicht 13 in Kontakt.

Wenn bei dem dargestellten erfindungsgemäßen Testträger durch externen mechanischen Druck die Deckfolie 14 nach unten in Richtung auf die Basisschicht 2 gedrückt wird, so kommt nicht nur die dritte Testschicht 13 in Kontakt mit der zweiten Testschicht 10, sondern wenn die Deckfolie weit genug heruntergedrückt wird, berührt ihr der ersten Testschicht zugewandtes Ende 14 b von oben das Schalterelement 11 und drückt dieses ebenfalls herunter auf die zweite Testschicht 10, so daß die erste Testschicht und die zweite Testschicht in einem einen Flüssigkeitsaustausch ermöglichenden Kontakt zueinander stehen. Dies ist die zweite Position des Schalterelements 11 (Schalter geschlossen), die in Figur 2 dargestellt ist.

Zur Durchführung einer Analyse wird ein Tropfen von beispielsweise 30 µl Blut auf die Erythrozytenabtrennschicht 7 (durch das Schutznetz 6 hindurch) aufgebracht. Das Plasma durchdringt die Erythrozytenabtrennschicht 7, wobei die Erythrozyten zurückbleiben, so daß reines Plasma in die erste Testschicht 8 gelangt. Wegen des Spaltes 12 ist ein weitere Übertritt des Plasmas in die zweite Testschicht nicht möglich, d. h. die Flüssigkeit bleibt in einer präzise vorbestimmbaren Art und Weise solange in der ersten Testschicht 8 stehen, bis durch externen Druck das Schalterelement 11 nach unten gegen die zweite Testschicht 10 gedrückt wird und damit der Spalt zwischen der ersten und zweiten Testschicht durch das kapillaraktive Material des Schalterelements 11 überbrückt wird. Die Flüssigkeit kann nun in die Testschicht 10 übertreten.

In der Testschicht 10 bleibt die Flüssigkeit wiederum für einen präzise vorbestimmbaren Zeitraum stehen, bis die klappenförmige Deckfolie 14 heruntergedrückt wird, so daß die dritte Testschicht 13 die zweite Testschicht 10 kontaktiert und somit eine weitere Reaktionsstufe des Tests ablaufen kann.

Bevorzugt wird das Schalterelement 11 wie oben erwähnt indirekt durch Herunterdrücken der Deckfolie 14 gegen die zweite Testschicht 10 betätigt. In der dargestellten Ausführungsform ist zu diesem Zweck die Deckfolie 14 erheblich länger als die dritte Testschicht 13 und erstreckt sich so weit in Richtung auf die erste Testschicht 8, daß sie in der in Fig. 2 dargestellten Position das Schalterelement überlappt. Dies ist insbesondere zweckmäßig, wenn der erfindungsgemäße Testträger im System mit einem entsprechenden Auswertegerät benutzt wird, welches ein entsprechendes bewegliches Teil aufweist, mit dem sich die Deckfolie 14 nach unten drücken läßt. Ein einziges bewegliches Teil des Geräts kann also über die Deckfolie 14 sowohl das Schalterlement 11 als auch die dritte Testschicht 13 gegen die zweite Testschicht 10 drücken. Ein solches Gerät ist in der Europäischen Patentanmeldung mit der Publikationsnummer 129 220 beschrieben. Dort wird als bewegliches Bauteil zum Andrücken der Meßkopf eines entsprechenden Reflexionsphotometers verwendet. Dieser ist in Figure 1 und 2 lediglich andeutungsweise dargestellt und mit 16 bezeichnet. Der Meßkopf 16 hat ein Meßfenster 16 a, durch das die reflexionsphotometrische Messung erfolgt. Die Deckfolie 14 ist bevorzugt durchsichtig um die photometrische Auswertung der Farbreaktion in der darunterliegenden Schicht zu ermöglichen.

Bei der in Figure 1 dargestellten Ausführungsform der Erfindung ist es besonders vorteilhaft, wenn das Schalterelement 11 aus einem Material besteht, welches im feuchten Zustand auf der zweiten Testschicht 10 haftet, sobald beide einmal durch Druck in Kontakt zueinander gebracht worden sind. Dadurch kann mit nur einem Betätigungselement (beispielsweise dem Meßkopf 16) des Auswertegerätes sehr einfach ein dreistufiger Reaktionsablauf erreicht werden. Wird die klappenartige Deckfolie 14 zum ersten Mal heruntergedrückt, so drückt ihr Ende 14 b das Schalterelement 11 in dieser Phase gegen die zweite Testschicht 10. Da das Schalterelement 11 in dieser Phase von der Probe befeuchtet ist, haftet es an der Testschicht 10. Der Flüssigkeitskontakt zwischen der ersten Testschicht 8 und der zweiten Testschicht 10 bleibt deswegen auch bestehen, wenn der Meßkopf 16 wieder angehoben wird. Da bei diesem ersten Andrücken der Deckfolie 14 die zweite Testschicht 10 noch trocken ist, findet kein Flüssigkeitsübertritt in die dritte Testschicht statt. Erst wenn eine zweite Reaktionszeit abgelaufen ist, in der die Flüssigkeit die zweite Testschicht 10 vollständig gefüllt hat und die in dieser Schicht vorgesehene Reaktion abgelaufen ist, wird der Meßkopf 16 erneut abgesenkt, so daß die Deckfolie 14 und die dritte Testschicht 13 wiederum gegen die zweite Testschicht 10 gedrückt werden. Da diese nun naß ist, tritt die Flüssigkeit in die dritte Testschicht über und die für diese Testschicht vorgesehene Reaktion kann ablaufen.

Das Material des Schalterelements 11 ist für seine zuverlässige Funktion von erheblicher Bedeutung. Einerseits muß es im trockenen Zustand ausreichend standfest sein, daß ein zu früher Kontakt mit der zweiten Testschicht 10 verhindert wird. Andererseits muß es im angefeuchteten Zustand ausreichend flexibel und kontaktfähig sein, um im nach unten gedrückten Zustand zuverlässig an der Testschicht 10 haften zu können, damit der Flüssigkeitstransport auf voller Breite der Testschicht gleichmäßig möglich ist. Überraschenderweise erfüllt eine Vielzahl von Materialien diese Anforderungen. Insbesondere haben sich dünne Papiere, Vliese unterschiedlicher Zusammensetzung, dünne Gewebe und dünne Schichten aus porösen Kunststoffen bewährt.

Besonders bevorzugt ist das erfindungsgemäße Testelement für die Durchführung immunologischer Bestimmungen eingerichtet. Soll beispielsweise ein in der Probe enthaltenes Antigen bestimmt werden, so befindet sich in der ersten Testschicht 8 ein enzym-markierter in der Probenflüssigkeit löslicher Antikörper für dieses Antigen. Er wird durch die Probe während einer ersten Reaktionszeit gelöst und mit dieser inkubiert, so daß das Antigen mit dem Antikörper spezifisch bindet. Nach diesem Vorgant enthält die Testschicht 8 Komplexe aus Probenantigen und enzymmarkiertem Antikörper sowie nicht gebundenen enzymmarkierten Antikörper.

Nach Herunterdrücken des Schalterelementes 11 treten diese Bestandteile mit der Probenflüssigkeit in die zweite Testschicht 10 über, in der sich in trägerfixierter Form ein Antigen für den Antikörper aus der ersten Testschicht befindet. Während einer zweiten Reaktionszeit wird der nicht komplexierte Antikörper an das trägerfixierte Antigen gebunden. Die Komplexe dagegen bleiben frei beweglich.

Auf der dritten Testschicht 13 befindet sich ein Substrat für das Enzym. Wird diese Testschicht zur Einleitung eines dritten Reaktionsschrittes nach unten gegen die zweite Testschicht gedrückt, so treten die frei beweglichen enzymmarkierten Antigen-Antikörper-Komplexe in die substrathaltige Testschicht 13 über. Das Enzym katalysiert eine Reaktion des Substrats, welche in bekannter Weise zu einem Nachweissignal, insbesondere zu einem Farbumschlag der Schicht 13 führt.

Der beschriebene Ablauf eines immunologischen Tests ist als sogenanter IEMA-Test für analytische Bestimmungen bekannt. Zur Bestimmung eines Antikörpers in der Probe kann es ebenfalls eingesetzt werden, wobei dann im zuvor beschriebenen Reaktionsablauf jeweils Antikörper und Antigen zu vertauschen sind. Dieses Testprinzip konnte bisher nicht mit der gewünschten Genauigkeit auf Testträgern durchgeführt werden, weil es voraussetzt, daß die verschiedenen Reaktionsstufen sauber getrennt voneinander ablaufen und erst nach Ablauf der jeweiligen Reaktionsstufe der Übertritt in die Testschicht möglich ist, in der sich die Reaktionsbestandteile der nächsten Reaktionsstufe befinden. Eine derartige Bestimmung ist deswegen ein sehr gutes Beispiel für die besondere Vorteilhaftigkeit des erfindungsgemäß möglich gewordenen mehrstufigen Testablaufes auf einem Testträger.

Figure 3 zeigt eine Ausführungsform, bei der das Schalterelement 17 kein integraler Bestandteil einer der Testschichten ist. Es wird aus einer Schicht kapillaraktiven Materials gebildet, die am Ende der Deckfolie 14 dergestalt befestigt ist, daß sie den Spalt 12 zwischen den Testschichten 8 und 10 überlappt, wenn die Deckfolie 14 nach unter gegen die Testschichten gedrückt wird. Das Schalterelement kann an der Deckfolie so fest befestigt sein, daß es mit dieser wieder von den Testschichten abhebt, wenn kein Druck mehr auf die Deckfolie ausgeübt wird. Bevorzugt ist es jedoch nur leicht, z. B. mit einem wasserlöslichen Kleber, an der Deckfolie 14 befestigt und besteht aus einem im feuchten Zustand an den Testschichten haftenden Material. In diesem Fall bleibt das Schalterelement 17 in Kontakt mit den Testschichten, auch wenn es nicht mehr von oben angedrückt wird und deswegen die Deckfolie 14 aufgrund ihrer Eingenelastizität wieder von den Testschichten und dem Schalterelement abhebt.

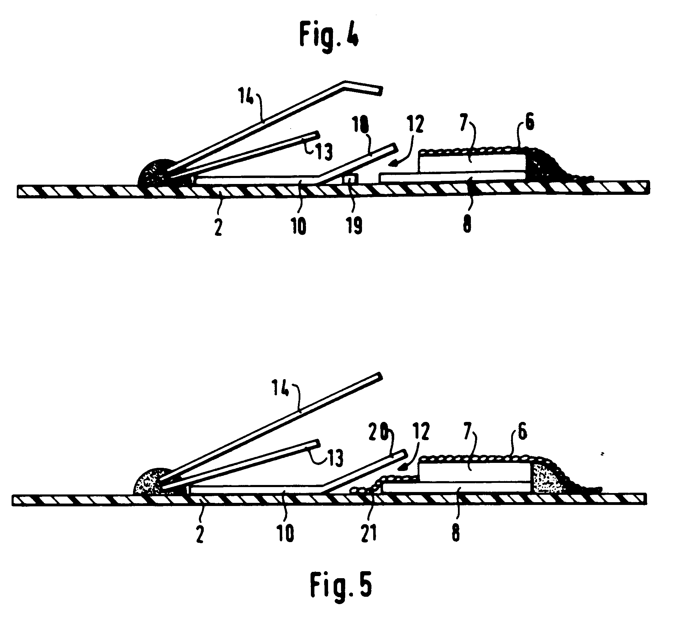

Figure 4 zeigt einen Testträger, bei dem das Schalterelement 18 integraler Bestandteil der zweiten Testschicht 10 ist. Außerdem ist ein zusätzliches Stützelement 19 zwischen dem Schalterelement 18 und der Basisschicht 2 vorgesehen. Dadurch wird besonders sichergestellt, daß das Schalterelement 18 erst dann in Kontakt mit der ersten Testschicht 8 tritt, wenn es von externem mechanischem Druck nach unten gedrückt wird.

Bei der in Figure 5 dargestellten Ausführungsform des Testträgers ist zwischen dem Schalterelement 20, welches in diesem Fall ein integraler Bestandteil der Testschicht 10 ist, und der Testschicht 8 eine hydrophobierte Netz- oder Gewebeschicht 21 angeordnet. Eine derartige Konstruktion erweist sich in Fällen als günstig, bei denen erst bei einem erhöhten mechanischem Druck auf das Schalterelement 20 ein Flüssigkeitsübergang von der ersten Testschicht 8 auf die zweite Testschicht 10 erreicht werden soll.

Beispiel 1:

Teststreifen zur Messung von N-Acetyl-beta-D-Glucosaminidase (NAG).

Ein Teststreifen gemäß Figure 1 wird aufgebaut:

Testschicht 8: 6 × 8 mm

Langfaserpapier (Schoeller und Hoesch, Gernsbach, Bundersrepublik Deutschland) wird 0,2 m Citrat-Puffer pH 4,9 getränkt.

Testschicht 10: 6 × 10 mm

Teebeutelpapier 212 (Schoeller und Hoesch, Gernsbach, Bundesrepublik Deutschland) wird mit einer Lösung von p-Nitrophenyl-N-acetylglucosaminid in 0,1 m Citratpuffer pH 4,9 getränkt.

Testschicht 13: 6 × 8 mm

Nylongewebe 20 HC (Schweizer Seidengazefabrik, Thal, Schweiz) wird getränkt mit einer wässrigen Kaliumcarbonat-Lösung.

Meßvorgang:

30 $\mu$l NAG-haltige Probelösung werden dosiert. Ein Aufenthalt von 90 sec in Reagenzienträger 7 und 8 dient der für eine enzymatische Reaktion nötigen Temperierung und der Einstellung des für die NAG nötigen sauren pH. Nach Andruck der Deckfolie 14 beginnt eine 2-minütige Reaktion in Testschicht 10. Bei erneutem Andruck der Deckfolie 14 wird umgepuffert. Nun kann das proportional zur NAG-Menge freigesetzte p-Nitrophenolat remissionsphotometrisch gemessen werden.

Beispiel 2:

Teststreifen zur Messung von Theophyllin in Blut.

Eins Teststreifen gemäß Figur 4 wird aufgebaut:

Testschicht 8: 6 × 8 mm

Glasfaserpapier P 300 (Binzer, Hatzfeld, Bundesrepublik Deutschland) wird getränkt mit einer Lösung von $F_{ab}$-beta-Galaktosidase-Konjugat gegen Theophyllin.

Testschicht 10: 6 × 11 mm

Durch Immunpräzipitation gemäß EP-A-185372 mit Anti-Fc (Schaf)-Antikörper wurde in einem CS-104-Cellulose/Polyester-Mischvlies ein IgG(Schaf)-theophyllin-Konjugat (Polyhapten) immobilisiert.

Testschicht 13: 6 × 8 mm

Teebeutelpapier 212 (Schoeller und Hoesch, Gernsbach, Bundesrepublik Deutschland) wird getränkt mit einer Lösung von Chlorphenolrot-beta-galaktosid in PBS-Puffer.

Meßvorgang:

30 $\mu$l theophyllinhaltigen Blutes werden auf die Erythrozytenabtrennschicht 7 pipettiert. Nach 3 Minuten erster Reaktionszeit im temperierten Meßgerät wird die Deckfolie 14 kurz angedrückt und betätigt den Schalter durch Druck auf das Schalterelement 18. Der folgende Flüssigkeitstransport (Chromatographie) entlang der Testschicht 10 dauert 1 Minute. Nun wird die Deckfolie 14 endgültig angedrückt und die proportional zum Theophyllingehalt noch freie $F_{ab}$-Galaktosidase-Menge durch remissionsphotometrische Messung bestimmt.

Beispiel 3:

Teststreifen zur Messung von Creatinkinase in Blut unter Hemmung der Myokinase.

Ein Teststreifen gemäß Figure 5 wird aufgebaut:

Testschicht 8: 6 × 8 mm

Multifiles Nylongewebe 4F (Schweizer Seidengazefabrik, Thal, Schweiz) wird getränkt mit einer Lösung von Diadenosinpentaphosphat und Adenosinmonophosphat.

Testschicht 10: 6 × 11 mm

Mischvlies Lutrabond 3670 (Faserprodukte Lahnstein, Lahnstein, Bundesrepublik Deutschland) wird getränkt mit einer Lösung von N-Acetylcystein und Creatinphosphat.

Testschicht 13: 6 × 8 mm

Teebeutelpapier 212 (Schoeller und Hoesch, Gernsbach, Bundesrepublik Deutschland) wird getränkt mit einer Lösung von Adenosindiphosphat, Glycerin, Glycerokinase, Peroxidase, Glycerin-3-phosphatoxidase und ABTS (Azino-di-[3-äthyl-benzthiazolin-sulfonat-6)]. Hydrophobes Gewebe 21 NY 150 HC (Schweizer Seidengazefabrik, Thal, Schweiz).

Meßvorgang:

30 μl CK-haltigen Blutes werden auf die Erythrozytenabtrennschicht 7 pipettiert. Während 60 Sekunden erster Reaktionszeit im temperierten Meßgerät findet auf Reagenzienträger 1 die Myokinase-Hemmung statt. Nach Andruck der Deckfolie 14 wird die hydrophobe Barriere überwunden, und in Testschicht 10 wird die CK aktiviert. Nach erneutem Andruck der Klappe findet der remissionsphotometrische Nachweis der CK statt.

**Patentansprüche**

1. Testträger (1) zur analytischen Bestimmung eines Bestandteils einer Körperflüssigkeit mit einer Basisschicht (2) und mindestens zwei flachen im Ausgangszustand des Testträgers vor Durchführung einer Bestimmung voneinander getrennten, jedoch durch externe Manipulation in Kontakt zueinander bringbaren Testschichten, dadurch gekennzeichnet, daß

eine erste Testschicht (8) und eine zweite Testschicht (10) im wesentlichen nebeneinander im Ausgangszustand durch einen Spalt getrennt auf der Basisschicht angeordnet sind, und

ein Schalterelement (11, 17, 18 20) vorgesehen ist, welches aus einem kapillaraktiven Material besteht, welches so dimensioniert ist, daß es den Spalt (12) überbrücken kann und welches so gelagert und angeordnet ist, daß es in einer ersten Position mindestens einer der Testschichten (8, 10) nicht kontaktiert und daß es durch externen Druck in eine zweite Position gebracht werden kann, in der es beide Testschichten derartig kontaktiert, daß ein Flüssigkeitaustausch zwischen den Testschichten möglich ist.

2. Testträger nach Anspruch 1, dadurch gekennzeichnet, daß eine biegbare Deckfolie (14) vorhanden ist, welche eine der Testschichten (10) klappenartig überspannt, wobei sie auf der von der anderen Testschicht abgewandten Seite an der Basisschicht befestigt ist und auf der anderen Seite das Schalterelement (11, 17, 18, 20) zumindest teilweise abdeckt, wenn sie durch externen Druck in Kontakt mit der Schicht, welche sie überspannt, gebracht wird.

3. Testträger nach Anspruch 2, dadurch gekennzeichnet, daß das Schalterelement (17) an der Deckfolie befestigt ist.

4. Testträger nach Anspruch 2, dadurch gekennzeichnet, daß zwischen der Deckfolie (14) und der von ihr überspannten Testschicht (10) eine dritte Testschicht (13) derartig angeordnet ist, daß sie nur durch externen Druck auf die Deckfolie (14) in einen einen Flüssigkeitsaustausch ermöglichenden Kontakt mit der von der Deckfolie (14) überspannten Testschicht (10) gebracht wird.

5. Testträger nach Anspruch 1, dadurch gekennzeichnet, daß die Materialien der Testschicht (8,10) und des Schalterelements (11, 17, 18, 20) zumindest an den sich kontaktierenden Oberflächen so aufeinander abgestimmt sind, daß das Schalterelement in von der Probenflüssigkeit angefeuchtetem Zustand an der Testschicht haftet (8, 10), nachdem es in die zweite Position gebracht wurde.

6. Testträger nach Anspruch 1, dadurch gekennzeichnet, daß das Schalterelement (11, 17, 18, 20) im Ausgangszustand in einer einen Flüssigkeitsaustausch ermöglichenden Verbindung mit einer der Testschichten (8, 10) steht und daß das Schalterelement so angeordnet ist, daß es diejenige Testschicht, mit der es im Ausgangszustand nicht in Verbindung steht, teilweise überlappt, ohne sie zu berühren.

7. Testträger nach Anspruch 6, dadurch gekennzeichnet, daß das Schalterelement (11, 18, 20) integraler Bestandteil der Testschicht ist, mit der es in einer einen Flüssigkeitsaustausch ermöglichenden Verbindung steht.

8. Testträger nach Anspruch 1, dadurch gekennzeichnet, daß zwischen den Testschichten (8,10) eine Abstützung (19) für das Schalterelement vorgesehen ist.

9. Testträger nach Anspruch 1, dadurch gekennzeichnet, daß eine hydrophobierte Netz- oder Gewebeschicht (21) im Bereich des die Testschichten trennenden Spaltes (12) so angeordnet ist, daß sie in der zweiten Position zwischen dem Schalterelement (20) und mindestens einer der Testschichten (8) liegt, so daß der den Flüssigkeitsaustausch ermöglichenden Kontakt erst durch erhöhten externen Druck auf das Schalterelement (20) möglich ist.

## Claims

1. Test carrier (1) for the analytical determination of a component of a body fluid with a base layer (2) and at least two planar test layers which, in the initial state of the test carrier, before carrying out of a determination, are separate from one another but can be brought into contact with one another by external manipulation, characterised in that a first test layer (8) and a second test layer (10) are arranged on the base layer essentially next to one another but separated in the initial state by a gap, a contact element (11, 17, 18, 20) is provided which consists of a capillary-active material which is so dimensioned that it can bridge the gap (12) and which is so mounted and arranged that, in a first position, it cannot contact at least one of the test layers (8, 10) and that, by external pressure, it can be brought into a second position in which it contacts both test layers in such a manner that a liquid exchange between the test layers is possible.

2. Test carrier according to claim 1, characterised in that a bendable covering film (14) is present which bridges one of the test layers (10) flap-like, whereby it is fixed on to the base layer on the side facing away from the other test layer, and, on the other side, at least partly covers the contact element (11,17,18,20) when, by external pressure, it is brought into contact with the layer which it bridges.

3. Test carrier according to claim 2, characterised in that the contact element (17) is fixed on to the covering film.

4. Test carrier according to claim 2, characterised in that, between the covering film (14) and the test layer (10) bridged by it, a third test layer (13) is arranged in such a manner that it is only brought by external pressure on the covering film (14) into a contact with the test layer (10) bridged by the covering film (14), making possible a liquid exchange.

5. Test carrier according to claim 1, characterised in that the materials of the test layers (8, 10) and of the contact element (11, 17, 18, 20) are so adjusted to one another at least on the contacting surfaces that the contact element, in a state moistened by the sample liquid, adheres to the test layers (8, 10) after it has been brought into the second position.

6. Test carrier according to claim 1, characterised in that the contact element (11, 17, 18, 20), in the initial state, stands in connection with one of the test layers (8, 10), making possible a liquid exchange and that the contact element is so arranged that it partly overlaps that test layer with which, in the initial state, it is not in connection, without touching it.

7. Test carrier according to claim 6, characterised in that the contact element (11, 17, 18, 20) is an integral component of the test layer with which it stands in connection making possible a liquid exchange.

8. Test carrier according to claim 1, characterised in that, between the test layers (8, 10), there is provided a support (19) for the contact element.

9. Test carrier according to claim 1, characterised in that a hydrophobed mesh or fabric layer (21) is so arranged in the region of the gap (12) separating the test layers that, in the second position, it lies between the contact element (20) and at least one of the test layers (8) so that the contact making possible the liquid exchange is only possible by increased external pressure on the contact element (20).

## Revendications

1. Support de test pour la détermination par analyse de la composition d'un liquide corporel, comportant une couche de base (2) et au moins deux couches de test plates, séparées entre elles dans l'état d'origine avant d'avoir effectué une détermination, mais que l'on peut mettre au contact l'une avec l'autre par une manipulation externe, caractérisé en ce qu'
    une première couche de test (8) et une deuxième couche de test (10) sont disposées essentiellement l'une à côté de l'autre sur la couche de base (2) dans l'état d'origine, séparées par un intervalle, et en ce qu'

un organe de raccordement (11, 17, 18, 20), constitué d'un matériau à action capillaire, qui est dimensionné de façon à pouvoir effectuer un pontage de l'intervalle (12) et qui est placé et orienté de telle façon que, dans une première position, il n'est pas en contact avec au moins l'une des couches de test (8, 10), et que, par une pression extérieure, il peut être placé dans une deuxième position dans laquelle les deux couches de test (8, 10) sont en contact de façon à ce qu'un échange de liquide soit possible entre les couches de test.

2. Support de test suivant la revendication 1, caractérisé en ce qu'il y a une feuille de couverture (14) souple, qui réalise un pontage, à la façon d'un volet, avec l'une des couches de test (10), cette feuille étant fixée à la couche de base (2) du côté opposé à l'autre couche de test, et recouvrant, de l'autre côté, au moins en partie, l'organe de raccordement (11, 17, 18, 20), quand elle est mise en contact, par une pression extérieure, avec la couche qu'elle recouvre.

3. Support de test suivant la revendication 2, caractérisé en ce que l'organe de raccordement (17) est fixé à la feuille de couverture.

4. Support de test suivant la revendication 2, caractérisé en ce qu'entre la feuille de couverture (14) et la couche de test (10) pontée par elle, une troisième couche de test (13) est disposée de telle façon que, seulement par une pression extérieure sur la feuille de couverture (14), elle réalise un contact, permettant un échange de liquide, avec la couche de test (10) pontée par la feuille de couverture (14).

5. Support de test suivant la revendication 1, caractérisé en ce que les matières de la couche de test (8, 10) et de l'organe de raccordement (11, 17, 18, 20) sont, au moins aux surfaces extérieures en contact, définies les unes par rapport aux autres de telle façon que l'organe de raccordement, lorsqu'il est mouillé par le liquide à tester, adhère à la couche de test (8, 10), après qu'il a été placé dans la deuxième position.

6. Support de test suivant la revendication 1, caractérisé en ce que l'organe de raccordement (11, 17, 18, 20), dans l'état d'origine, se trouve relié de façon à permettre un échange de liquide avec l'une des couches de test (8, 10) et en ce que l'organe de raccordement est disposé de telle façon que, sans la toucher,il recouvre en partie la couche de test avec laquelle il n'est pas relié dans l'état d'origine.

7. Support de test suivant la revendication 6, caractérisé en ce que l'organe de raccordement (11, 17, 18, 20) est une partie intégrante de la couche de test avec laquelle il se trouve relié de façon à permettre un échange de liquide.

8. Support de test suivant la revendication 1, caractérisé en ce qu'entre les couches de test (8, 10), il est prévu un appui (19) pour l'organe de raccordement.

9. Support de test suivant la revendication 1, caractérisé en ce que, dans la zone de l'intervalle (12) séparant les couches de test, une couche hydrophobe réticulaire ou de tissu (21) est disposée de telle façon que, dans la deuxième position, elle se trouve entre l'organe de raccordement (20) et au moins l'une des couches de test (8), de telle façon que le contact permettant l'échange de liquide soit seulement possible quand on exerce une pression extérieure plus forte sur l'organe de raccordement (20).

Fig.1

Fig. 2

Fig. 3

## Fig. 4

## Fig. 5